(19) Europäisches Patentamt
European Patent Office
Office européen des brevets

(11) Veröffentlichungsnummer: **0 054 946**
**B1**

# (12) EUROPÄISCHE PATENTSCHRIFT

(45) Veröffentlichungstag der Patentschrift: **15.08.84**

(51) Int. Cl.³: **C 07 D237/22**

(21) Anmeldenummer: **81110620.2**

(22) Anmeldetag: **19.12.81**

## (54) Basisch substituierte Pyridazine, ihre Herstellung und ihre Verwendung.

(30) Priorität: **22.12.80 DE 3048487**

(43) Veröffentlichungstag der Anmeldung: **30.06.82 Patentblatt 82/26**

(45) Bekanntmachung des Hinweises auf die Patenterteilung: **15.08.84 Patentblatt 84/33**

(84) Benannte Vertragsstaaten: **AT BE CH DE FR GB IT LI NL SE**

(56) Entgegenhaltungen:
**DE-A- 2 819 629**
**DE-A- 3 023 369**
**GB-A- 2 010 261**

(73) Patentinhaber: **CASSELLA Aktiengesellschaft**
**Hanauer Landstrasse 526**
**D-6000 Frankfurt am Main 61 (DE)**

(72) Erfinder: **Raabe, Thomas, Dr.**
**Schillerstrasse 5**
**D-6451 Rodenbach (DE)**
Erfinder: **Bohn, Helmut, Dr.**
**Kranzbergring 11**
**D-6369 Schöneck (DE)**
Erfinder: **Martorana, Piero A., Dr.**
**Kaiser-Friedrich-Promenade 108**
**D-6380 Bad Homburg v.d. H. (DE)**
Erfinder: **Nitz, Rolf-Eberhard, Dr.**
**Heinrich-Bingemer-Weg 64**
**D-6000 Frankfurt am Main 60 (DE)**

(74) Vertreter: **Urbach, Hans-Georg, Dr. et al**
**Hanauer Landstrasse 526**
**D-6000 Frankfurt am Main 61 (DE)**

**EP 0 054 946 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft pharmakologisch wertvolle basisch substituierte Pyridazine der allgemeinen Formel I

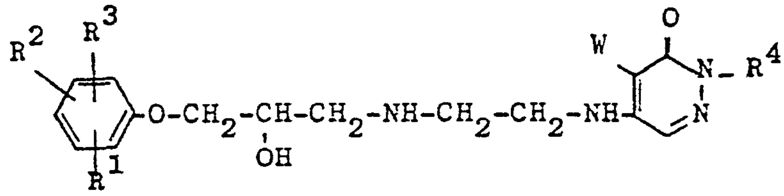

(I)

worin $R^1$, $R^2$ und $R^3$ unabhängig voneinander Wasserstoff, Halogen, Hydroxy, Nitro, Trifluormethyl, Alkyl, Alkoxyalkyl, Alkenyl, Alkinyl, Cycloalkyl, Cycloalkenyl, Phenyl, Alkoxy, Hydroxyalkoxy, Alkoxy-alkoxy, Alkenyloxy, Alkinyloxy, Cycloalkoxy, Phenalkoxy, Alkanoyl, Acylamino, einen —NH—CO—$R^9$-Rest, wobei $R^9$ für Morpholino, Piperidino oder 1-Pyrrolidinyl steht, einen gegebenenfalls substituierten Ureido-Rest, $R^4$ Wasserstoff oder niederes Alkyl und W Wasserstoff, Chlor oder Brom bedeuten, sowie deren Säureadditionssalze.

Die Verbindungen der allgemeinen Formel I besitzen in der Alkanolamin-Seitenkette ein asymmetrisches C-Atom und können daher in racemischen und optisch aktiven Formen existieren. Im Rahmen der vorliegenden Erfindung werden unter den Verbindungen der allgemeinen Formel I auch mögliche Stereoisomere und optisch aktive Verbindungen und Mischungen davon, insbesondere das Racemat, verstanden.

Die für $R^1$, $R^2$ und $R^3$ genannten Substituenten besitzen neben Wasserstoff, Halogen, Hydroxy, Nitro, Trifluormethyl und Phenyl insbesondere folgende Bedeutungen :

Alkyl mit 1 bis 8, vorzugsweise 1 bis 4 C-Atomen, z. B. Methyl, Ethyl, Propyl, Isopropyl, n-Butyl, Isobutyl, tert.-Butyl, sec.-Butyl, n-Pentyl, Isopentyl, Neopentyl, tert.-Pentyl, n-Hexyl, Isohexyl, n-Heptyl, n-Octyl ; Alkenyl mit bis zu 6, vorzugsweise 3 oder 4, C-Atomen, beispielsweise Vinyl, Allyl, 1-Propenyl, Isopropenyl, Methallyl, Crotyl, 2-Pentenyl, 2-Hexenyl ;

Alkinyl mit bis zu 6, vorzugsweise 3 oder 4, C-Atomen, z. B. Propargyl ;

Cycloalkyl mit einer Ringgröße von 5 bis 8, vorzugsweise 5 oder 6, C-Atomen, z. B. Cyclopentyl und Cyclohexyl ;

Cycloalkenyl mit einer Ringgröße von 5 bis 8, vorzugsweise 5 oder 6, C-Atomen, z. B. Cyclopentenyl ;

Alkoxy mit 1 bis 8 C-Atomen, z. B. Methoxy, Ethoxy, Propoxy, Butoxy, Isopropoxy, Isohexyloxy, n-Heptyloxy, n-Octyloxy, Pentyloxy, Isopropoxy, Isohexyloxy, n-Heptyloxy, n-Octyloxy, Pentyloxy; Cycloalkoxy mit einer Ringgröße von 5 bis 8 C-Atomen im Cycloalkylteil, vorzugsweise Cyclopentyloxy und Cyclohexyloxy ;

Alkenyloxy mit bis zu 6, vorzugsweise 3 oder 4, C-Atomen, z. B. Allyloxy, Methallyloxy, Crotyloxy, 2-Hexenyloxy ;

Alkinyloxy mit bis zu 6, vorzugsweise 3 oder 4, C-Atomen, beispielsweise Propargyloxy ;

Alkanoyl mit 1 bis 6 C-Atomen, beispielsweise Formyl, Acetyl, Propionyl, Butyryl, Isobutyryl, Valeryl, Isovaleryl, Pivaloyl, Alkoxyalkoxy mit insgesamt bis zu 8 C-Atomen, wobei der Alkoxyalkoxyrest von der Form $R^6O—R^5O—$ ist und $R^5$ einen Alkylenrest mit 2 bis 7 C-Atomen, $R^6$ einen Alkylrest mit 1 bis 6 C-Atomen darstellt und die Reste $R^5$ und/oder $R^6$ bei 3 und mehr C-Atomen auch verzweigt sein können. Beispiele für geeignete Alkoxyalkoxyreste sind : 2-Methoxy-ethoxy, 2-Ethoxy-ethoxy, 3-Methoxy-n-propoxy, 2-Methoxy-n-propoxy, 4-Methoxy-n-butoxy, 3-Ethoxy-n-propoxy, 2-Ethoxy-n-propoxy, 4-Ethoxy-n-butoxy, 3-Ethoxy-n-butoxy, 2-Ethoxy-n-butoxy, 2,2-Dimethyl-2-ethoxy-ethoxy, 3-(n-Propoxy)-n-propoxy, 2-(n-Propoxy)-n-propoxy, 3-(iso-Propoxy)-n-propoxy, 2-(iso-Propoxy)-n-propoxy, 2-(n-Propoxy)-ethoxy, 2-(iso-Propoxy)-ethoxy, 4-(n-Propoxy)-n-butoxy, 3-(n-Propoxy)-n-butoxy, 2-(n-Butoxy)-ethoxy, 2-(sec.-Butoxy)-ethoxy, 2-(tert.-Butoxy)-ethoxy, 3-(n-Butoxy) n-propoxy, 2-(n-Butoxy)-n-propoxy, 3-(iso-Butoxy)-n-propoxy, 3-(sec.-Butoxy)-n-propoxy, 3-(tert.-Butoxy)-n-propoxy, 4-(n-Butoxy)-n-butoxy, 3-(n-Butoxy)-n-butoxy, 2-(n-butoxy)-n-butoxy, 4-(iso-Butoxy)-n-butoxy, 3-(iso-Butoxy)-n-butoxy, 2-(sec.-Butoxy)-n-butoxy, 2,2-Dimethyl-2-(n-butoxy)-ethoxy, 2-(n-Butoxy)-1-methyl-ethoxy, 2-(iso-Butoxy)-2-methyl-ethoxy, 5-Methoxy-n-pentyloxy, 4-Methoxy-n-pentyloxy, 3-Methoxy-n-pentyloxy, 5-Ethoxy-n-pentyloxy, 4-Ethoxy-n-pentyloxy, 3-Ethoxy-n-pentyloxy, 5-(n-Propoxy)-n-pentyloxy, 5-(iso-Propoxy)-n-pentyloxy, 6-Methoxy-n-hexyloxy, 5-Methoxy-n-hexyloxy, 4-Methoxy-n-hexyloxy, 6-Ethoxy-n-hexyloxy, 3-Ethoxy-n-hexyloxy, 7-Methoxy-n-heptyloxy ;

Alkoxyalkoyl mit 2 bis 6 C-Atomen, wobei der Alkoxyalkylrest von der Form $R^8O—R^7—$ ist und $R^8$ einen Alkylrest und $R^7$ einen Alkylenrest darstellen, wobei die Reste $R^8$ und/oder $R^7$, falls sie mehr als 3 C-Atome enthalten, auch verzweigt sein können, beispielsweise : Methoxy-methyl, Ethoxy-methyl, n-Propoxy-methyl, iso-Propoxy-methyl, n-Butoxy-methyl, n-Pentyloxy-methyl, 2-Methoxy-ethyl, 2-Ethoxyethyl, 2-n-Propoxy-ethyl, 2-iso-Propoxy-ethyl, 2-n-Butoxy-ethyl, 3-Methoxy-n-propyl, 3-Ethoxy-n-propyl, 3-n-Propoxy-n-propyl, 2-Methyloxy-2-methyl-ethyl, 2-Ethoxy-1-methyl-ethyl, 2-n-Propoxy-2-methyl-ethyl, 2-iso-Propoxy-1-methyl-ethyl, 4-Methoxy-n-butyl, 4-Ethoxy-n-butyl, 5-Methoxy-n-pentyl ;

Hydroxyalkoxy mit 2 bis 6 C-Atomen, beispielsweise 2-Hydroxy-ethoxy, 3-Hydroxy-n-propoxy, 4-Hydroxy-n-butoxy, 3-Hydroxy-n-butoxy, 5-Hydroxy-n-pentyloxy, 4-Hydroxy-n-hexyloxy, 2-Hydroxy-n-hexyloxy, 2-Hydroxy-n-propoxy ;

Phenalkyloxy, z. B. Phenethyloxy, insbesondere aber Benzyloxy ; Ureido oder ein Ureidorest, der in 3-Stellung durch Alkyl mit 1 bis 6 C-Atomen, vorzugsweise 1 bis 4-C-Atomen, Alkenyl mit 3 bis 6 C-Atomen oder Cycloalkyl mit 5 oder 6 C-Atomen substituiert ist, wobei bei Alkyl- und/oder Alkenylsubstitution auch eine Disubstitution möglich ist. Beispiele für geeignete Ureidoreste sind Ureido, 3-Methylureido, 3-Ethylureido, 3-Propylureido, 3-Isopropylureido, 3-Allylureido, 3-Cyclopentylureido, 3-Cyclohexylureido, 3,3-Dimethylureido, 3,3-Diäthylureido ;

Als Halogen kommen beispielsweise Fluor, Chlor oder Brom in Betracht ;

Einen —NH—CO—$R^9$-Rest, wobei $R^9$ für Morpholino, Piperidino oder 1-Pyrrolidinyl steht.

Bei dem für $R^1$ und/oder $R^2$ und/oder $R^3$ stehenden Acylamino-Rest wird unter dem Begriff Acyl der sich von einer aromatischen aromatisch-aliphatischen oder aliphatischen Carbonsäure ableitenden aryl-, arylalkyl- oder alkylsubstituierte Carbonylrest mit bis zu 11 C-Atomen verstanden. Geeignete Acylaminoreste sind z. B. Acetamino, Propionylamino, Butyrylamino, Benzoylamino, α- und β-Naphthoylamino, Phenylacetylamino, bevorzugt werden Acetamino und Benzoylamino.

Der Substituent $R^4$ kann neben Wasserstoff insbesondere für einen Alkylrest mit 1 bis 4 C-Atomen stehen, beispielsweise für Methyl, Ethyl, n-Propyl, Isopropyl, n-Butyl, Isobutyl.

Normalerweise werden Verbindungen der allgemeinen Formel I bzw. deren Säureadditionssalze bevorzugt, bei denen $R^4$ für Wasserstoff steht oder nur einer der Substituenten $R^1$, $R^2$ oder $R^3$ ungleich Wasserstoff ist oder $R^1$, $R^2$ oder $R^3$ eine Hydroxy, Alkoxyalkoxy-, Alkoxy-, Alkoxyalkyl- oder Hydroxyalkoxyrest oder Halogen ist. Besonders bevorzugt sind solche Verbindungen, die zwei oder mehrere der vorgenannten bevorzugten Eigenschaften besitzen, also bei denen $R^4$ Wasserstoff und einer der Substituenten $R^1$, $R^2$ oder $R^3$ ein Hydroxy-, Alkoxyalkoxy-, Alkoxy-, Alkoxyalkyl oder Hydroxy-alkoxyrest oder Halogen bedeutet und die beiden anderen Wasserstoff sind. Für $R^1$, $R^2$ und $R^3$ werden normalerweise solche Reste bevorzugt, die keine Asymmetriezentren enthalten.

Zur Bildung von Säureadditionssalzen mit den Verbindungen der allgemeinen Formel I sind anorganische und organische Säuren geeignet. Geeignete Säuren sind beispielsweise Chlorwasserstoff, Bromwasserstoff, Naphthalindisulfonsäure (1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Citronen- oder Adipinsäure. Pharmazeutisch annehmbare Säureadditionsalze werden bevorzugt. Die Säureadditionsalze können wie üblich durch Vereinigung der Komponnenten, zweckmäßigerweise in einem geeigneten Verdünnungs bzw. Dispergiermittel, erhalten werden.

Zur Herstellung der Verbindungen der allgemeinen Formel I wird eine Verbindung der allgemeinen Formel V

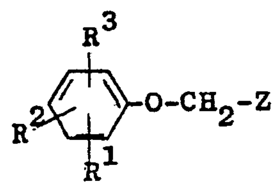

(V)

wobei Z für

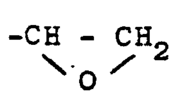

oder

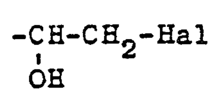

steht und Hal ein Halogenatom, insbesondere Chlor oder Brom bedeutet, mit einer Verbindung der allgemeinen Formel IV

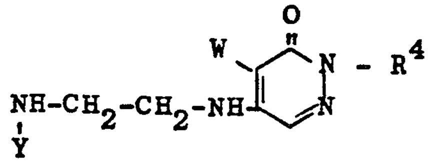

(VI)

wobei Y für Wasserstoff oder einen hydrogenolytisch abspaltbaren Rest steht, umgesetzt und aus der erhaltenen Verbindung ein vorhandener hydrogenolytisch abspaltbarer Rest anschliessend durch Hydrogenolyse abgespalten und die erhaltene Verbindung gegebenenfalls mit einer Säure zu einem Säureadditionssalz umgesetzt.

Zahlreiche hydrogenolytisch abspaltbare Reste sind bereits seit langem bekannt und haben sich insbesondere auf dem Gebiet der Peptidsynthese sehr gut bewährt. Sehr instruktive Arbeiten und Übersichten wurden veröffentlicht insbesondere von R. A. Boissonnas et G. Preitner, Helvetica Chimica Acta Bd. 36, S. 875 (1953) und R. A. Boissonnas, Advances in Organic Chemistry Bd. 3, S. 159 ff. (1963) mit zahlreichen Hinweisen auf Primär-Literatur.

Beispiele für hydrogenolytisch abspaltbare Reste sind insbesondere im Phenylkern durch Methyl, Methoxy, Chlor oder Phenylazo substituiertes Benzyl, Carbobenzoxy oder Carbophenyloxy sowie Carboallyloxy.

Anstelle einer einheitlichen Verbindung der allgemeinen Formel V kann auch ein Gemisch einer Verbindung der allgemeinen Formel II mit einer im Phenylkern gleich substituierten Verbindung der allgemeinen Formel III

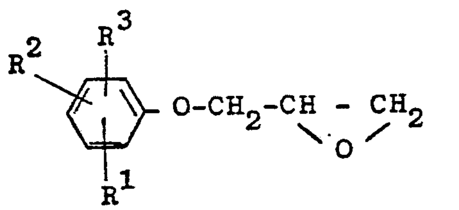

(II)

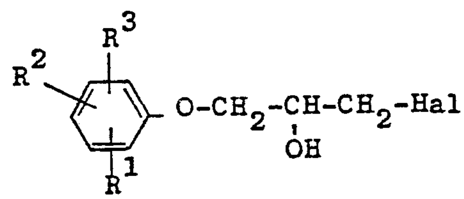

(III)

wobei in Formel III Hal ein Halogenatom, insbesondere Chlor oder Brom bedeutet, eingesetzt werden.

Die Umsetzung der Verbindungen der allgemeinen Formeln IV und V wird normalerweise in einem geeigneten Lösungs- oder Dispersionsmittel durchgeführt, in dem die Reaktionspartner gelöst bzw. suspendiert werden. Solche Lösungs- oder Dispersionsmittel sind z. B. Wasser, aromatische Kohlenwasserstoffe, wie z. B. Benzol, Toluol, Xylol ; Ketone, wie z. B. Aceton, Methylethylketon ; halogenierte Kohlenwasserstoffe, wie z. B. Chloroform, Tetrachlorkohlenstoff, Chlorbenzol, Methylenchlorid, Äther, wie z. B. Tetrahydrofuran und Dioxan ; Sulfoxide, wie z. B. Dimethylsulfoxid ; tertiäre Säureamide, wie z. B. Dimethylformamid und N-Methylpyrrolidon. Als Lösungsmittel werden insbesondere polare Lösungsmittel, wie z. B. Alkohole, verwandt. Geeignete Alkohole sind z. B. Methanol, Ethanol, Isopropanol, tert.-Butanol usw. Alkohole mit 1 bis 4 C-Atomen werden bevorzugt. Die Reaktion wird bei Temperaturen von 20 °C bis zur Rückflußtemperatur des verwendeten Lösungs- oder Dispergiermittels durchgeführt. Die Reaktion läuft häufig bei Temperaturen von 60 bis 100 °C ab. Es kann zweckmäßig sein, die Ausgangsverbindung der allgemeinen Formel IV in einem bis zu 10fach oder gegebenenfalls noch höheren molaren Überschuß einzusetzen und/oder die Reaktionskomponente der allgemeinen Formeln II und III in gelöster bzw. suspendierter Form zu der gelösten bzw. suspendierten Reaktionskomponente der allgemeinen Formel IV zuzugeben. Das Molverhältnis zwischen den Verbindungen der allgemeinen Formel II bzw. III und IV kann daher 1 : 1 bis 1 : 10 und gegebenenfalls noch mehr betragen. Bei der Anwesenheit einer Verbindung der allgemeinen Formel III kann die Umsetzung auch in Gegenwart von säurebindenden Mitteln, wie Pottasche, Soda, Triethylamin usw. durchgeführt werden. Ohne säurebindende Mittel erhält man dann gewöhnlich die Hydrohalogenide für den Fall, daß Y = Wasserstoff ist, die Hydrohalogenide der Verbindungen der allgemeinen Formel I.

Die Umsetzung von Epoxiden bzw. Halohydrinen mit Aminogruppen enthaltenden Verbindungen und die Bedingungen, unter denen diese Reaktion zweckmäßig durchgeführt wird, sind an sich bekannt. Eine zusammenfassende Darstellung findet sich in Houben-Weyl, 4. Auflage (1965), Bd. XI/1 Seiten 24-74 sowie 314-326.

Bei der Umsetzung der Verbindungen der allgemeinen Formeln IV und V erhält man für den Fall, daß in der allgemeinen Formel IV Y für einen hydrogenolytisch abspaltbaren Rest, beispielsweise für den Benzyl- oder Carbobenzoxyrest, steht, zunächst Verbindungen der allgemeinen Formel Ia

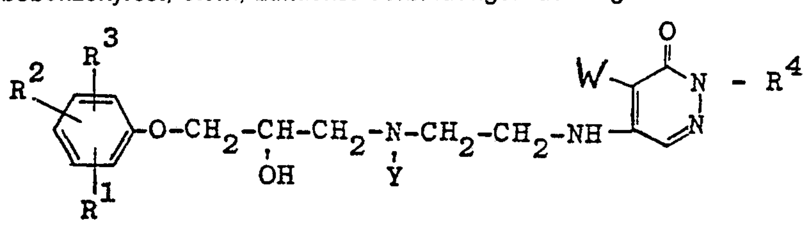

(Ia)

worin Y für einen hydrogenolytisch abspaltbaren Rest steht. Man erhält die Hydrohalogenide der Verbindungen der allgemeinen Formel Ia beim Einsatz von Verbindungen der allgemeinen Formel III und bei Abwesenheit von säurebindenden Mitteln. Die Überführung der Verbindungen der allgemeinen Formel Ia bzw. deren Hydrohalogenide in die Verbindungen der allgemeinen Formel I erfolgt durch hydrogenolytische Abspaltung des Restes Y nach üblichen Verfahren. Hierbei wird die Verbindung der allgemeinen Formel Ia bzw. deren Hydrohalogenide in einem geeigneten Lösungsmittel, wie z. B. einem Alkanol, Äther oder Kohlenwasserstoff, wie Ethanol, Dioxan, Toluol, Xylol etc., gelöst oder suspendiert und in Gegenwart eines geeigneten Katalysators, wie z. B. Palladium/Kohle, bei Temperaturen von Raumtemperatur (20 °C) bis zur Rückflußtemperatur des verwendeten Lösungsmittels mit Wasserstoff behandelt. Nach dem Absaugen des Katalysators kann die Verbindung der allgemeinen Formel I isoliert werden. Die hydrogenolytische Abspaltung des Restes Y findet zumeist schon bei Raumtemperatur (20 °C) statt.

Die Herstellung der Ausgangsverbindungen der allgemeinen Formel IV kann durch Umsetzung von Verbindungen der allgemeinen Formel VI

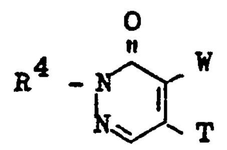

(VI)

wobei W die oben angegebenen Bedeutungen hat unt T für Chlor oder Brom steht, mit einer Verbindung der allgemeinen Formel VII

$$H_2N—CH_2—CH_2—NH—X \qquad (VII)$$

erfolgen, wobei X einen hydrolytisch abspaltbaren Rest einer Schutzgruppe oder den Rest Y (= Wasserstoff oder einen hydrogenolytisch abspaltbaren Rest) bedeutet. Ein hydrolytisch abspaltbarer Rest ist z. B. der Acetylrest oder ein anderer Acylrest, d. h. ein von einer aliphatischen, aromatischen oder araliphatischen Carbonsäure durch OH-Abspaltung sich ableitender Rest. Ein hydrogenolytisch abspaltbarer Rest ist, wie bereits erwähnt, z. B. der Benzyl- oder Carbobenzoxy-Rest. Die Umsetzung der Verbindungen der allgemeinen Formel VI mit den Verbindungen der allgemeinen Formel VII wird normalerweise in einem geeigneten Lösungs- oder Dispergiermittel durchgeführt, in dem die Reaktionspartner gelöst bzw. suspendiert werden, wie z. B. Benzol, Toluol, Xylol, Chloroform, Methylenchlorid, Tetrachlorkohlenstoff, Diethyläther, Dioxan, Tetrahydrofuran, Dimethylsulfoxid, Aceton, Methylethylketon, Dimethylformamid, N-Methylpyrrolidon usw. Das Molverhältnis zwischen den Verbindungen der allgemeinen Formel VI und VII kann 1 : 1 bis 1 : 10 und gegebenenfalls noch mehr betragen. Die Reaktion kann bei Raumtemperatur ausgeführt werden, oder sie kann durch die Anwendung von Wärme beschleunigt oder zu Ende geführt werden, beispielsweise durch Erhitzen auf eine Temperatur von 80 bis 110 °C.

Steht bei der Umsetzung eine Verbindung der allgemeinen Formel VI mit einer Verbindung der allgemeinen Formel VII X für Wasserstoff, so wird die Verbindung der allgemeinen Formel VII zweckmäßigerweise im Überschuß eingesetzt, in einigen Fällen auch als Lösungsmittel. Steht bei der Umsetzung einer Verbindung der allgemeinen Formel VI mit einer Verbindung der allgemeinen Formel VII X für eine hydrolytisch oder hydrogenolytisch abspaltbare Schutzgruppe und werden beide Reaktionskomponenten in gleichmolaren Mengen eingesetzt, so wird die Umsetzung zweckmäßigerweise in Gegenwart von säurebindenden Mitteln, wie z. B. Pottasche, Soda, Triethylamin usw. durchgeführt.

Steht bei der Umsetzung einer Verbindung der allgemeinen Formel VI mit einer Verbindung der allgemeinen Formel VII X für eine Schutzgruppe, dann entsteht zuerst eine Verbindung der allgemeinen Formel VIII

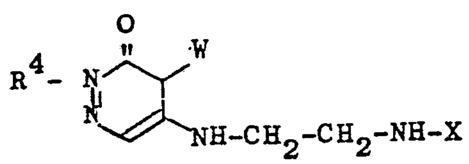

(VIII)

aus der man durch Abspaltung der Schutzgruppe X mit den üblichen Methoden, z. B. wenn X für ein Acylradikal steht, durch Hydrolyse, oder, falls X für einen hydrogenolytisch abspaltbaren Rest steht, durch Hydrogenolyse zu einer Verbindung der allgemeinen Formel IV kommt, in der Y für H steht.

Zur Herstellung der Verbindungen der allgemeinen Formel I kann man auch Pyridazine der allgemeinen Formel VI

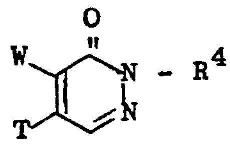

(VI)

worin $R^4$, W und T die oben genannten Bedeutungen haben, mit einem Diamin der allgemeinen Formel IX

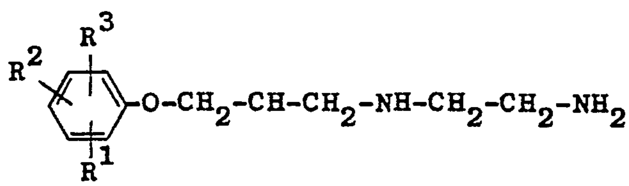

(IX)

umsetzen und die erhaltene Verbindung gegebenenfalls mit einer Säure zu einem Säureadditionssalz umsetzen.

Die Umsetzung einer Verbindung der allgemeinen Formel VI mit einer Verbindung der allgemeinen Formel IX wird normalerweise in einem geeigneten Lösungs- oder Dispergiermittel durchgeführt, in der die Reaktionspartner gelöst, bzw. suspendiert werden, wie z. B. Benzol, Toluol, Xylol, Chloroform, Methylenchlorid, Tetrachlorkohlenstoff, Chlorbenzol, Dioxan, Äther, Tetrahydrofuran, Wasser, Dimethylsulfoxid, Dimethylformamid, N-Methylpyrrolidon usw.

Die Reaktion kann bei Raumtemperatur ausgeführt werden, oder sie kann durch die Anwendung von Wärme beschleunigt oder zu Ende geführt werden, beispielsweise durch Erhitzen auf eine Temperatur zwischen 80 und 120 °C. Das Molverhältnis zwischen den Verbindungen der allgemeinen Formeln VI und IX kann 1 : 1 bis 1 : 10 und gegebenenfalls noch mehr betragen. Setzt man gleichmolare Mengen der Verbindungen der allgemeinen Formeln VI und IX ein, so ist es empfehlenswert, in Gegenwart von mindestens äquimolaren Mengen eines säurebindenden Mittels, wie z. B. Pottasche, Soda, Triethylamin usw. zu arbeiten. Ohne säurebindende Mittel erhält man dann gewöhnlich die Hydrohalogenide der Verbindungen der allgemeinen Formel I.

Auch die Umsetzung von Halogenpyridazinen mit Aminogruppen enthaltenden Verbindungen und die zweckmäßigen Reaktionsbedingungen sind an sich ebenfalls bekannt. Eine Übersicht und zahlreiche Literaturzitate finden sich in R. Elderfield, Heterocyclic Compounds (1957) Bd. 6, Seite 130. Weitere Druckschriften betreffend diese Umsetzung sind z. B. D. P. 579,391 [CA *27*, 4631 (1933)] und Meier, Ringier Druey, Helv. Chim. Acta, Bd. 37, Seiten 510 und 523 (1954).

Zur Herstellung der Ausgangsdiamine der allgemeinen Formel IX kann man eine Verbindung der allgemeinen Formel II oder III oder ein Gemisch einer Verbindung der allgemeinen Formel II mit einer im Phenylkern gleichsubstituierten Verbindung der allgemeinen Formel III mit einer Verbindung der allgemeinen Formel VII (wobei X Wasserstoff oder eine hydrolytisch abspaltbare Schutzgruppe, wie einen Acetyl- oder anderen Acylrest bedeutet) umsetzen. Diese Umsetzung wird normalerweise in einem geeigneten Lösungs- oder Dispergiermittel durchgeführt, indem die Reaktionspartner gelöst bzw. suspendiert werden. Solche Lösungs- oder Dispergiermittel sind z. B. Wasser, aromatische Kohlenwasserstoffe wie z. B. Benzol, Toluol, Xylol ; Ketone, wie z. B. Aceton, Methylethylketon ; halogenierte Kohlenwasserstoffe, wie z. B., Chloroform, Tetrachlorkohlenstoff, Chlorbenzol, Metylenchlorid ; Äther, wie z. B. Tetrahydrofuran und Dioxan ; Sulfoxide, wie z. B. Dimethylsulfoxide ; tertiäre Säureamide, wie z. B. Dimethylformamid und N-Methylpyrrolidon. Als Lösungsmittel werden insbesondere polare Lösungsmittel, wie z. B. Alkohole, verwandt. Geeignete Alkohole sind z. B. Methanol, Ethanol, Isopropanol, tert.-Butanol usw.

Die Reaktion wird bei Temperaturen von 20 °C bis zur Rückflußtemperatur des verwendeten Lösungs- oder Dispergiermittels durchgeführt. Die Reaktion läuft häufig bei Temperaturen von 60 bis 100 °C ab. Es kann zweckmäßig sein, die Ausgangsverbindungen der allgemeinen Formel VII in einem bis zu 10fach oder gegebenenfalls noch höheren molaren Überschuß einzusetzen und/oder die Reaktionskomponente der allgemeinen Formel II und III in gelöster bzw. suspendierter Form zu der gelösten bzw. suspendierten Reaktionskomponente der allgemeinen Formel VII zuzugeben. Das Molverhältnis zwischen den Verbindungen der allgemeinen Formel II bzw. III und VII kann 1 : 1 bis 1 : 10 und gegebenenfalls noch mehr betragen. Bei der Anwesenheit einer Verbindung der allgemeinen Formel III kann die Umsetzung auch in Gegenwart von säurebindenden Mitteln, wie Pottasche, Soda usw. durchgeführt werden. Ohne säurebindende Mittel erhält man dann gewöhnlich die Hydrohalogenide der Verbindungen der allgemeinen Formel IX.

Steht bei der Umsetzung der Verbindungen der allgemeinen Formel II oder III mit einer Verbindung

der allgemeinen Formel VII X für eine Schutzgruppe, dann entsteht zuerst eine Verbindung der allgemeinen Formel X

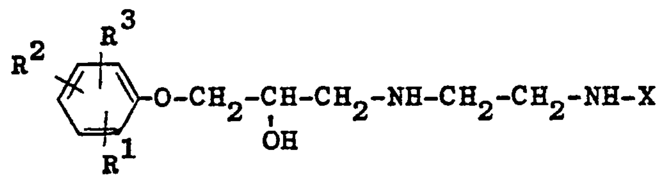

(X)

aus der man durch Abspaltung der Schutzgruppe X nach den üblichen Methoden, z. B. wenn X für ein Acylradikal steht, durch Hydrolyse zu einer Verbindung der allgemeinen Formel IX kommt.

Die vorstehenden Herstellungsverfahren sind bevorzugt für erfindungsgemäße Verbindungen, in denen W Chlor oder Brom bedeutet.

Erfindungsgemäße Verbindungen der Formel I, in der W Wasserstoff ist, können prinzipiell nach den oben beschriebenen Herstellungsverfahren erhalten werden. Häufig sind jedoch Ausgangsverbindungen der Formeln IV oder VI, in denen W Wasserstoff bedeutet, nur umständlich durch mehrstufige Synthese zugänglich (vergl. z. B. Elderfield, Heterocyclic Compounds, Bd. 6, Seite 130 (1957)). Es ist daher vorteilhaft, erfindungsgemäße Verbindungen der Formel I, in denen W Wasserstoff bedeutet, aus erfindungsgemäßen Verbindungen der Formel I, in denen W Chlor oder Brom ist, durch Austausch des für W stehenden Halogenatoms gegen Wasserstoff herzustellen. Der selektive Austausch eines am Pyridazinon-Ring gebundenen, für W stehenden Halogenatoms erfolgt auf einfache Weise in glatter Reaktion durch Hydrierung. In der Regel wird hierbei eine Lösung oder Dispersion der Substanz in einem Lösungs- oder Dispergiermittel in Gegenwart eines Hydrierkatalysators, beispielsweise von Raney-Nickel oder eines feinverteilten Platinmetalls auf einem geeigneten Träger, wie z. B. Palladium auf Kohle, mit Wasserstoff behandelt.

Besonders zweckmäßig als Lösungsmittel bzw. Dispergiermittel haben sich niedere Alkanole mit 1-4 C-Atomen, Wasser oder Wasser/Alkanolgemische erwiesen. Zum Abfangen des bei der Hydrierung entstehenden Halogenwasserstoffs wird die Reaktion in Gegenwart einer Base, wie z. B. eines tertiären Amins, MgO, Alkaliacetat oder Alkalihydroxid, durchgeführt.

Die Durchführung solcher Austauschreaktionen durch Hydrierung ist bereits eingehend untersucht und beschrieben worden, so z. B. in Houben-Weyl, 4. Auflage (1965) Bd. V/4, Seiten 773 ff sowie Bd. IV ic, Seiten 364 ff.

Anstelle von erfindungsgemäßen Verbindungen der Formel I, in denen W Chlor oder Brom ist, können zur Herstellung von erfindungsgemäßen Verbindungen der Formel I, in denen W Wasserstoff ist, auch Verbindungen der Formel

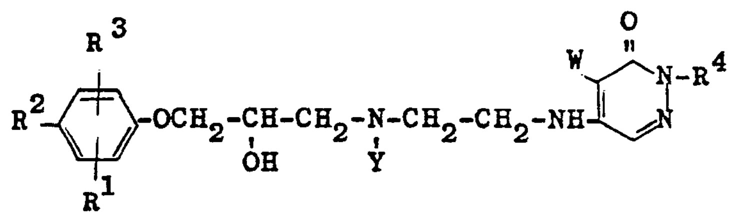

(Ia)

eingesetzt werden, worin W Chlor oder Brom und Y ein hydrogenolytisch abspaltbarer Rest ist und $R^1$, $R^2$, $R^3$ und $R^4$ die oben genannten Bedeutungen haben. Bei der Hydrierung dieser Verbindungen in der oben angegebenen, an sich bekannten Weise gelingt es, in ein und demselben Reaktionsschritt sowohl das für W stehende Halogenatom als auch den hydrogenolytisch abspaltbaren Rest Y durch Wasserstoff zu erstezen.

Verbindungen der Formel Ia werden wie oben ausgeführt bei der Umsetzung von Verbindungen der Formel V mit Verbindungen der Formel IV, in denen Y ein hydrogenolitisch abspaltbarer Rest ist, erhalten.

Die Ausgangsverbindungen der allgemeinen Formel VI sind entweder bekannt, oder sie können in bekannter Weise hergestellt werden. So lassen sich Ausgangsverbindungen, in denen W und T Chlor oder Brom sind, leicht aus den Hydrazinderivaten der allgemeinen Formel XI durch Umsetzung mit Mucochlorsäure, Mucobromsäure oder der entsprechenden Chlor-Brom-Verbindung herstellen (vergl. Katritzky, Boulton, Advances in Heterocyclic Chemistry (1968) Bd. 9, S. 235-236). Am Beispiel der Mucochlorsäure sei der Reaktions verlauf demonstriert :

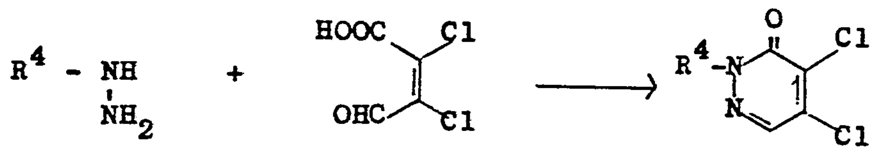

Ausgangsverbindungen der Formel VI, in denen W Wasserstoff bedeutet, können beispielsweise gemäß dem Reaktionsschema in R. Elderfield, Heterocyclic Compounds (1957), Bd. 6, Seite 130, ausgehend von 4,5 Dibrompyridazinon-(3) hergestellt werden.

Die Verbindungen der allgemeinen Formeln II und III können nach bekannten Methoden, z. B. durch Umsetzung des entsprechenden Phenols mit Epichlorhydrin, hergestellt werden.

Optisch aktive Formen der Alkylendiamine der allgemeinen Formel I können durch Trennung der entsprechenden racemischen Alkylendiamine der allgemeinen Formel I mittels üblicher Methoden erhalten werden, z. B. dadurch, daß man das Racemat einer Verbindung der allgemeinen Formel I mit einer optisch aktiven Säure umsetzt, hierauf eine fraktionierte Kristallisation des so erhaltenen diastereomeren Salzgemischs aus einem geeigneten Verdünnungs- oder Lösungsmittel, wie z. B. Ethanol, anschließt, und schließlich das optisch aktive Alkylendiamin mit einer Base aus dem Salz in Freiheit setzt. Optisch aktive Verbindungen der allgemeinen Formel I können auch dadurch erhalten werden, daß optisch aktive Ausgangsverbindungen III oder IX eingesetzt werden. Diese optisch aktiven Ausgangsverbindungen erhält man in bekannter Weise durch Racematspaltung aus den optisch inaktiven Verbindungen III oder IX.

Die erfindungsgemäßen Verbindungen der allgemeinen Formel I und deren Säureadditionssalze besitzen wertvolle pharmazeutische Eigenschaften. Insbesondere besitzen sie eine stark ausgeprägte β-adrenalytische Wirkung, die außerdem kardioselektiv ist, d. h., die Verbindungen besitzen einen größeren Grad von Spezifität bei der Blockierung der kardialen β-Rezeptoren als der peripheren β-Rezeptoren, z. B. der β-Rezeptoren im Bronchialmuskel. Darüber hinaus, besitzen sie zum Teil starke α-lytische, antiarrhythmische und blutdrucksenkende Wirkungen. Sie sind daher z. B. für die Behandlung oder Prophylaxe von Herzbeschwerden und Herzkrankheiten wie Angina pectoris und Herzarrhythmien geeignet, ferner zur Behandlung von Hypertension, ohne daß bei empfindlichen Patienten die Lunge angegriffen wird.

Die erfindungsgemäßen Verbindungen der allgemeinen Formel I sind in ihrer pharmazeutischen Wirkung den bekannten ähnlich gebauten Verbindungen der DE-A-2 819 629 überraschenderweise deutlich überlegen.

Die erfindungsgemäßen Pyridazine können daher am Menschen für sich allein, in Mischungen untereinander oder in pharmazeutischen Zubereitungen verabreicht werden, die als aktiven Bestandteil eine wirksame Dosis mindestens eines erfindungsgemäßen Pyridazins oder eines Säureadditionssalzes davon neben üblichen pharmazeutisch einwandfreien Träger- und Zusatzstoffen enthalten.

Geeignete Trägerstoffe sind z. B. Wasser, pflanzliche Öle, Stärke, Gelatine, Milchzucker, Magnesiumstearat, Wachse, Vaseline etc. Als Zusatzstoffe können z. B. Netzmittel, Sprengmittel, Konservierungsmittel etc. verwendet werden.

Die pharmazeutischen Präparate können in Form von z. B. Tabletten, Kapseln, wäßrigen oder öligen Lösungen oder Suspensionen, Emulsionen, injizierbaren wäßrigen oder öligen Lösungen oder Suspensionen, dispergierbaren Pulvern oder Aerosolmischungen vorliegen. Die pharmazeutischen Präparate können neben den Verbindungen der allgemeinen Formel I auch noch eine oder mehrere andere pharmazeutisch wirksame Substanzen, beispielsweise Beruhigungsmittel wie z. B. Luminal, Meprobamat, Chlorpromazine und Benzodiazepinsedativa, wie z. B. Diazepam oder Chlordiazepoxid ;

Vasodilatoren, wie z. B. Glyzerintrinitrat, Pentaerythrittetranitrat und Carbochromen ;

Diuretica, wie z. B. Chlorothiazid ;

das Herz tonisierende Mittel, wie z. B. Digitalis-Präparate ;

Hypotensionsmittel, wie z. B. Rauwolfia-Alkaloide und Guanethidin ;

Bronchodilatatoren und sympathomimethische Mittel, wie z. B. Isoprenalin, Osciprenalin, Adrenalin und Ephedrin ;

α-adrenergische Blockierungsmittel, wie z. B. Phentolamin ;

Kardialmembranstabilisierungsmittel (Antiarrythmika), wie z. B. Chinidin und Katecholamine, wie Noradrenalin, enthalten.

Die Herstellung der Verbindungen der allgemeinen Formel I wird an folgenden Beispielen näher erläutert :

Beispiel 1

4,9 g N-[3-(o-Chlorphenoxy)-2-hydroxy]-ethylendiamin

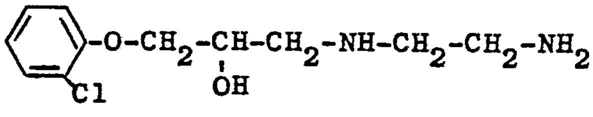

werden in 50 ml Ethanol gelöst. Dazu gibt man eine Lösung von 3,3 g 4,5-Dichlor-pyridazinon (3)

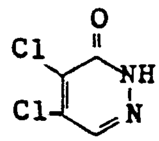

in 50 ml Ethanol und erhitzt die Mischung anschließend 12 Stunden am Rückfluß. Dann wird die Mischung i. Vak. eingeengt, der Rückstand mit wenig Essigester digeriert, vom Essigester abdekantiert und der Rückstand schließlich aus Ethanol umkristallisiert.

Man erhält so das N-[3-(o-Chlorphenoxy)-2-hydroxy-propyl]-N'-[4-chlor-3-oxo-pyridazyl(5)]-ethylendiamin-hydrochlorid

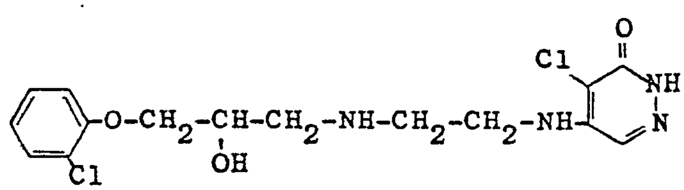 HCl

Fp. : 219 °C
Analyse : ($C_{15}H_{19}Cl_3N_4O_3$)
berechnet : C 44,0 H 4,6 Cl 26,0 N 13,7 O 11,7
gefunden : C 44,4 H 4,8 Cl 27,7 N 13,5 O 12,2
Ausbeute : 78 % d. Theorie.

Das als Ausgangsprodukt verwendete N-[3-(o-Chlorphenoxy)-2-hydroxypropyl]-ethylendiamin kann wie folgt hergestellt werden :

120 g Ethylendiamin werden in 150 ml Ethanol gelöst. Dazu gibt man eine Lösung von 20 g o-Chlorphenylglycid-äther

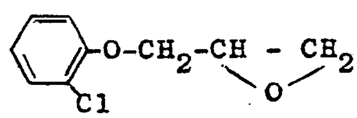

in 40 ml Ethanol und erhitzt die Mischung 20 Stunden am Rückfluß. Dann wird i. Vak. überschüssiges Ethylendiamin und Ethanol abdestilliert und der Rückstand anschließend i. Vak. destilliert.

Man erhält so das N-[3-(o-Chlorphenoxy)-2-hydroxy-propyl]-ethylendiamin als ein bei Kp 190 °C/0,4 mm destillierbares Öl. Das als Ausgangsprodukt benutzte 4,5-Dichlor-pyridazinon(3) kann durch Umsetzung von Mucochlorsäure mit Hydrazin in bekannter Weise (z. B. nach K. Dury, Angew. Chemie *77*, S. 282 (1965) hergestellt werden.

Beispiel 2

5,1 g N-[3-(o-Ethoxyphenoxy)-2-hydroxy-propyl]-ethylendiamin

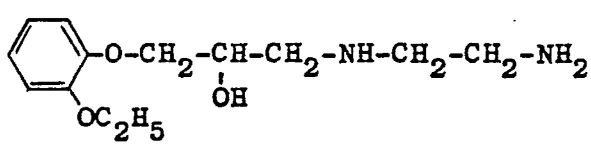

werden in 50 ml Toluol gelöst und 3 g Pottasche zugesetzt. Dann gibt man unter Rühren bei Raumtemperatur eine Lösung von 3,6 g 2-Methyl-4,5-dichlor-pyridazinon (3)

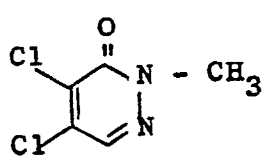

in 50 ml Toluol zu und erhitzt anschließend 17 Stunden unter Rühren am Rückfluß. Dann läßt man auf Raumtemperatur abkühlen, saugt von anorganischem Rückstand ab und engt das Filtrat i. Vak. ein. Das zurückbleibende Öl, das nach kurzer Zeit fest wird, wird aus Essigester umkristallisiert.

Man erhält so das N-[3-(o-Ethoxyphenoxy)-2-hydroxy-propyl]-N'-[2-methyl-3-oxo-4-chlor-pyridazyl(5)]-ethylendiamin

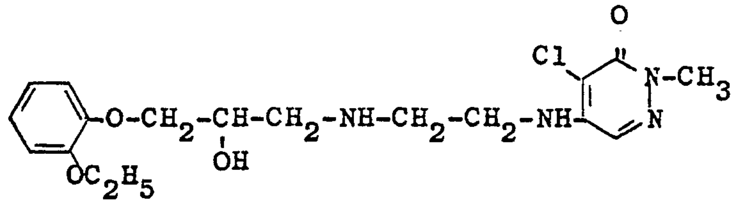

Fp. : 120 °C
Analyse : ($C_{18}H_{25}ClN_4O_4$)
berechnet : C 54,5 H 6,3 Cl 9,0 N 14,1 O 16,1
gefunden : C 54,7 H 6,3 Cl 9,1 N 13,8 O 16,4
Ausbeute : 84 % d. Theorie
Die Ausgangsprodukte können analog den in Beispiel 1 beschriebenen Verfahren hergestellt werden.

## Beispiel 3

3,8 g 2',4',6'-Trimethyl-phenylglycidäther

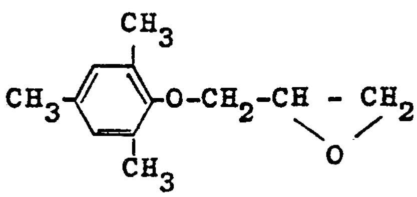

werden zusammen mit 7 g N-Benzyl-N'-[2-butyl-3-oxo-4-chlor-pyridazyl(5)]-ethylendiamin

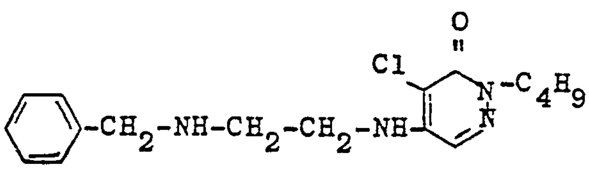

in 80 ml Ethanol 1 St. am Rückfluß erhitzt. Dann wird abgekühlt und die Lösung i. Vak. eingeengt.

Der Rückstand wird ohne Reinigung in 120 ml Dioxan gelöst und anschließend mit $H_2$ in Gegenwart von Pd/Kohle bei Raumtemperatur hydriert.

Dann wir abgesaugt, das Filtrat i. Vak. eingeengt und der Rückstand aus Ethanol umkristallisiert. Man erhält so das
N-[3-(2',4',6'-Trimethylphenoxy)-2-hydroxy-propyl]-N-[2-butyl-3-oxo-4-chlor-pyridazyl(5)]-ethylendiamin

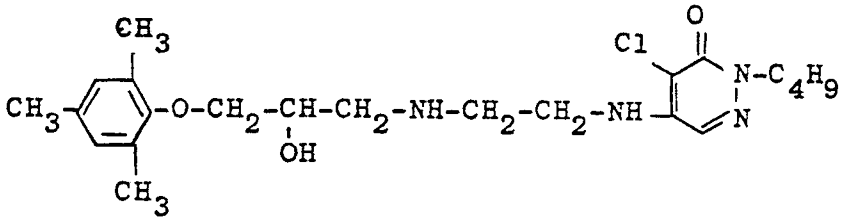

Fp. : 137 °C
Analyse : ($C_{22}H_{33}ClN_4O_3$)
berechnet : C 60,5 H 7,6 Cl 8,1 N 12,8 O 11,0
gefunden : C 60,2 H 7,4 Cl 8,3 N 12,7 O 11,3
Ausbeute : 68 % d. Theorie

Das als Ausgangsprodukt benutzte N-Benzyl-N'-[2-butyl-3-oxo-4-chlor-pyridazyl(5)]-ethylendiamin kann durch Umsetzung von 2-Butyl-4,5-dichlor-pyridazinon(3) mit Benzylethylendiamin in Gegenwart von 1 Mol Pottasche in siedendem Toluol analog Beispiel 2 erhalten werden.

Beispiel 4

5,85 g N-[3-(p-Pentyloxyphenoxy)-2-hydroxy-propyl]-ethylendiamin der Formel

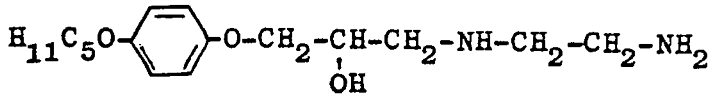

und 5,0 g 4,5-Dibrom-pyridazinon-(3) werden in 60 ml Ethanol 10 Std. am Rückfluß gekocht. Dann wird die erhaltene Lösung filtriert und eingeengt, wobei ein zähes Öl zurückbleibt. Dieser ölige Rückstand wird mit 100 ml Wasser und 5-10 ml Essigsäure-ethylester verrührt und mit wäßriger 2N-Sodalösung auf einen pH-Wert von 9,0 eingestellt. Man rührt diese Mischung, bis das Öl vollständig durchkristallisiert ist und saugt das erhaltene Festprodukt ab. Der Rückstand (5,4 g entsprechend 58 % d. Th.) wird aus Ethanol umkristallisiert. Man erhält 4,2 g N-[3-p-Pentyloxyphenoxy)-2-hydroxy-propyl]-N'-[3-oxy-4-brompyridazyl-(5)]-ethylendiamin der Formel

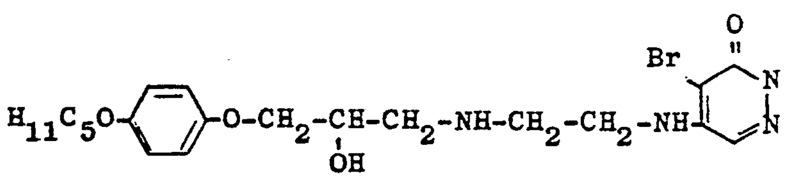

vom Schmelzpunkt 173-175 °C

Analyse : $C_{20}H_{29}BrN_4O_4$

| | | | | | |
|---|---|---|---|---|---|
| berechnet : | C 51,14 | H 6,2 | N 11,9 | O 13,6 | Br 17,0 |
| gefunden : | C 51,0 | H 6,3 | N 11,5 | O 13,9 | Br 16,8 |

Beispiel 5

4,13 g N-(3-Phenoxy-2-hydroxy-propyl)-ethylendiamin der Formel

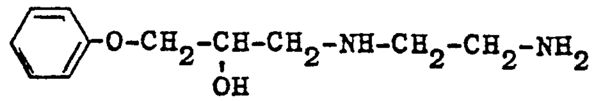

und 5,0 g 4,5-Dibrom-pyrazinon-(3) werden in 80 ml wasserfreiem Ethanol 30 Std. am Rückfluß gekocht. Man filtriert die erhaltene Lösung und dampft das Filtrat ein. Es hinterbleibt ein halbfester Rückstand, der mit 100 ml Wasser und 10 ml Essigsäure-ethylester verrührt wird. Die Mischung wird nun durch Zusatz von wäßriger 2N-Sodalösung auf pH 9,0 eingestellt, wobei das Reaktionsprodukt allmählich fest wird.

Das feste Produkt wird abgesaugt und aus Ethanol umkristallisiert. Man erhält so das N-(3-Phenoxy-2-hydroxy-propyl)-N'-[3-oxo-4-brompyridazyl-(5)]-ethylendiamin der Formel

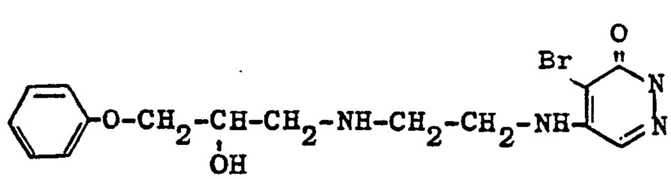

von Fp 165 °C in einer Ausbeute von 3,9 g, entsprechend 52 % d. Th.

Analyse : $C_{15}H_{19}BrN_4O_3$

| | | | | | |
|---|---|---|---|---|---|
| berechnet : | C 47,0 | H 5,0 | N 14,6 | O 12,5 | Br 20,9 |
| gefunden : | C 46,8 | H 4,7 | N 14,6 | O 12,9 | Br 20,8 |

Beispiel 6

3,0 g N-(3-Phenoxy-2-hydroxypropyl)-N'-[4-chlor-3-oxo-pyridazyl-(5)]-ethylendiamin-hydroxychlorid der Formel

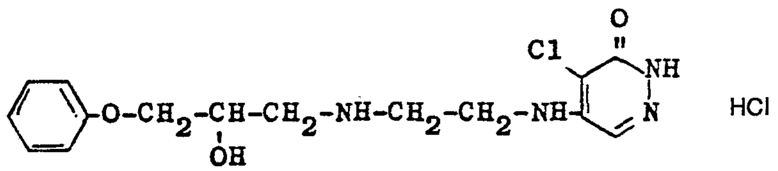

werden in 100 ml 5 gew.%iger Natronlauge gelöst, und die Lösung wird mit 0,3 g Palladium/Kohle 10 %ig versetzt. Dann wird bei Raumtemperatur in einer Schüttelente unter Normaldurck mit Wasserstoff hydriert, bis kein weiterer Wasserstoffverbrauch mehr stattfindet, und danach läßt man den Ansatz noch über Nacht bei Raumtemperatur stehen.

Die so erhaltene Reaktionsmischung wird filtriert, der im wesentlichen aus dem Katalysator bestehende Filterrückstand $R_1$ wird aufbewahrt, das Filtrat wird zur Trockenen eingedampft. Der Eindampfrückstand wird mit 50 ml Wasser gut durchgeknetet und die erhaltene Suspension abgesaugt. Man erhält so den Filterrückstand $R_2$.

Der Filterrückstand $R_1$ wird mit 50 ml Ethanol unter Erwärmen durchgeschüttelt und die Suspension vom Katalysator abfiltriert. Das Alkoholfiltrat wird eingedampft, der Eindampfrückstand mit dem Filterrückstand $R_2$ vereinigt und beide gemeinsam aus Wasser umkristallisiert. Man erhält 2,0 g (~ 82 % d. Th.).

N-(3-Phenoxy-2-hydroxypropyl)-N'-[3-oxopyridazyl-(5)]-ethylendiamin der Formel

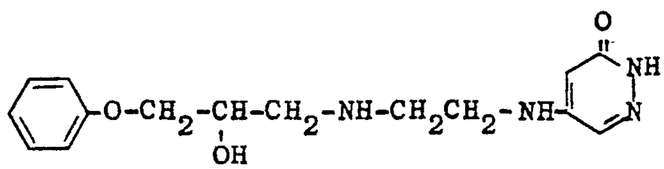

Schmelzpunkt 151-153 °C
Analyse : $C_{15}H_{20}N_4O_3$

| | | | | |
|---|---|---|---|---|
| berechnet : | C 59,18 | H 6,63 | N 18,41 | O 15,78 |
| gefunden : | C 59,1 | H 6,2 | N 18,4 | O 16,0 |

Beispiel 7

2 g N-[4-Chlor-3-oxopyridazyl-(5)]-ethylendiamin der Formel

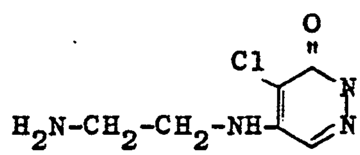

und

1,95 g o-Chlorphenyl-glycidether der Formel

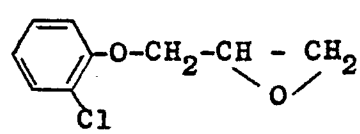

werden in 20 ml wasserfreiem Ethanol zunächst 24 Std. bei Raumtemperatur und dann noch 40 Std. unter Sieden am Rückflußkühler gerührt. Dann wird der Ansatz abgekühlt und der ausgeschiedene Niederschlag abgesaugt. Der Filterrückstand wird nochmals aus Ethanol umkristallisiert. Man erhält 3,1 g (~ 78,3 % d. Th.) N-[3-(o-Chlorphenoxy)-2-hydroxypropyl]-N'-[4-chlor-3-oxo-pyridazyl-(5)]-ethylendiamin der Formel

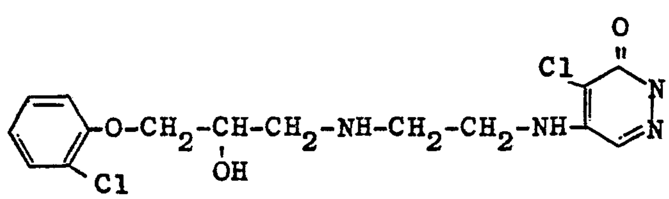

vom Schmelzpunkt 170-172 °C

Analyse : $C_{15}H_{18}Cl_2N_4O_3$

berechnet : C 48,24 H 4,86 Cl 19,02 N 15,01 O 12,86

gefunden : C 48,3 H 4,8 Cl 19,2 N 15,5 O 12,6

Das in obigem Beispiel eingesetzte N-[4-Chlor-3-oxo-pyridazyl-(5)]-ethylendiamin wurde wie folgt hergestellt :

Zu einer Lösung von 400 g Ethylendiamin in 100 ml absol. Ethanol wurden 11 g 4,5-Dichlor-pyridazinon-(3)-gegeben und die Mischung im Autoklaven 12 Std. auf 120 °C erhitzt.

Nach dem Abkühlen wurde die erhaltene Lösung zur Trockene eingedampft und der Rückstand aus Ethanol/Wasser umkristallisiert.

Ausbeute : 9 g (~ 71,6 % d. Th.).

Entsprechend den Beispielen 1-7 wurden die in der nachfolgenden Tabelle genannten Verbindungen hergestellt :

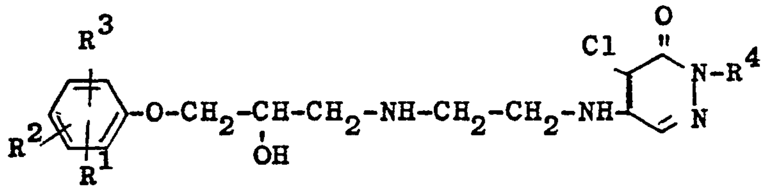

| $R^1$ | $R^2$ | $R^3$ | $R^4$ | Fp.: |
|---|---|---|---|---|
| $4-OC_4H_9$ | H | H | $-CH_3$ | 92°C |
| $2-OC_2H_5$ | H | H | H | 185°C (.HCl) |
| $2-OC_2H_5$ | H | H | $-C_2H_5$ | 83°C |
| $4-OCH_3$ | H | H | H | 213°C (.HCl) |
| $4-OC_5H_{11}$ | H | H | H | 223°C (.HCl) |
| $2-CH_3$ | H | H | H | 228°C (. HCl) |
| H | H | H | H | 198°C (. HCl) |
| $4-OC_4H_9$ | H | H | H | 210°C (. HCl) |
| $3-OCH_3$ | H | H | H | 213°C (. HCl) |
| $3-OCH_3$ | $4-OCH_3$ | $5-OCH_3$ | $-CH_3$ | 69°C |
| $2-OCH_3$ | $3-OCH_3$ | $4-OCH_3$ | $-C_3H_7$ | 199°C (. HCl) |
| 2-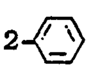 | H | H | H | 224°C (. HCl) |
| $2-OCH_2-CH=CH_2$ | H | H | $-CH_3$ | 111°C |
| 2-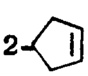 | H | H | $-C_2H_5$ | 109°C |
| $2-OCH_3$ | $6-OCH_3$ | H | H | 218°C (. HCl) |
| $4-NH-CO-CH_3$ | H | H | $-C_3H_7$ | 197°C (. HCl) |
| $2-O-CH_2-C\equiv CH$ | H | H | $-CH_3$ | 121°C |

(Fortsetzung)

| $R^1$ | $R^2$ | $R^3$ | $R^4$ | Fp.: |
|---|---|---|---|---|
| $4-NH-CO-NH_2$ | H | H | H | 113°C |
| 2-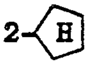 | H | H | H | 68°C |
| 4-NH-CO-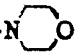 | H | H | $-C_2H_5$ | 219°C (.H Cl) |
| $4-CH_2-O-CH_3$ | H | H | H | 117°C |
| 4-C 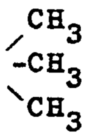 | H | H | $-C_4H_9$ | 71°C |
| $2-CO-CH_3$ | H | H | H | 193°C (. HCl) |
| $2-CH_2-OC_2H_5$ | H | H | $-CH_3$ | 211°C (. HCl) |
| $4-NH-CO-NH-C_2H_5$ | H | H | H | 217°C (. HCl) |
| 4-NH-CO-NH-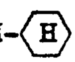 | H | H | H | 119°C |
| $4-NH-CO-NH-CH_2-CH=CH_2$ | H | H | $-C_3H_7$ | 231°C (. HCl) |
| 2-Cl | 6-Cl | H | $-C_3H_7$ | 101°C |
| $2-CH_3$ | $4-CH_3$ | H | $-CH_3$ | 74°C |
| $2-CH_3$ | $6-CH_3$ | H | H | 198°C (. HCl) |
| $H-OC_2H_4-OC_2H_5$ | H | H | $-C_2H_5$ | 206°C (. HCl) |
| $4-OCH_2-CH_2-OH$ | H | H | $-CH_3$ | 89°C |
| $4-C_2H_5$ | H | H | $-C_2H_5$ | 102°C |
| $2-OCH_3$ | $4-CH_2-CH=CH_2$ | H | $-CH_3$ | 107°C |
| $3-CF_3$ | H | H | H | 216°C (. HCl) |
| 4-OH | H | H | $-C_2H_5$ | 122°C |
| 2-Cl | 4-Cl | H | H | 199°C (. HCl) |
| $4-O-CH_2-$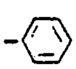 | H | H | $-C_2H_5$ | 204°C (. HCl) |
| $4-NO_2$ | H | H | H | 57°C |
| 2-Cl | 4-Cl | H | $-CH_3$ | 87°C |
| 4-Br | H | H | $-C_2H_5$ | 223°C (. HCl) |
| 2- H | H | H | H | 196°C (. HCl) |
| $2-CH_3$ | $4-NO_2$ | H | H | 112°C |
| $3-CH_2$ | 4-Cl | H | $-C_4H_9$ | 208°C (. HCl) |

| $R^1$ | $R^2$ | $R^3$ | $R^4$ | Fp.: |
|---|---|---|---|---|
| $2-CH_2-C\equiv CH$ | H | H | $-C_2H_5$ | 74°C |
| $2-CH_2$ | $3-CH_3$ | $5-CH_3$ | $-C_3H_7$ | 121°C |

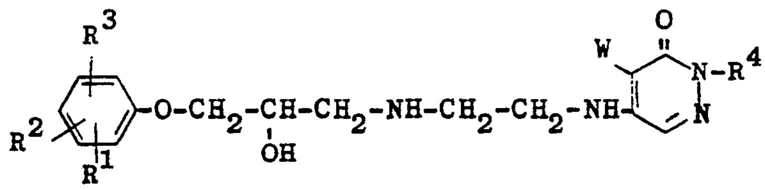

| $R^1$ | $R^2$ | $R^3$ | $R^4$ | W | Fp: |
|---|---|---|---|---|---|
| $4-OC_4H_9$ | H | H | H | Br | 221°C (. HCl) |
| $2-O-CH_2-CH=CH_2$ | H | H | H | Br | 166°C (. HCl) |
| 4-NH-CO-N‹morpholino›O | H | H | H | Br | 208°C (. HCl) |
| $2-O-CH_2-C\equiv CH$ | H | H | H | Br | 188°C (. HCl) |
| $2-CH_3$ | $4-CH_3$ | H | $CH_3$ | Br | 87°C |
| 2 Cl | H | H | H | Br | 159°C |
| $3-OCH_3$ | H | H | H | Br | 162°C |
| $2-OC_2H_5$ | H | H | H | Br | 200°C (. HCl) |
| 2-Cl | H | H | H | H | 143°C |
| $3-CH_3O$ | H | H | H | H | 149°C |
| $2-OC_2H_5$ | 4-Cl | H | H | H | 168°C |
| $2-O-CH_2-CH=CH_2$ | $4-OCH_3$ | H | H | H | 154°C |

Die wesentliche technische Überlegenheit der erfindungsgemäßen Verbindungen gegenüber einem typischen Vertreter bekannter Handelsprodukte gleicher Wirkungsrichtung und ähnlicher chemischer Struktur, dem Metoprolol, ergibt sich aus folgenden pharmakologischen Vergleichsdaten.

Strukturen der verglichenen Verbindungen

1. Erfindungsgemäß

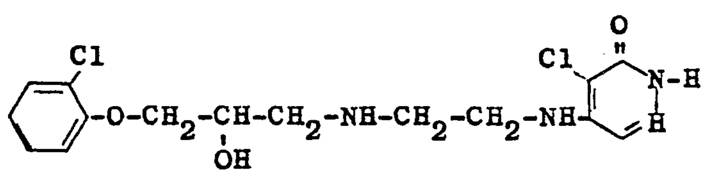

2. Metoprolol :

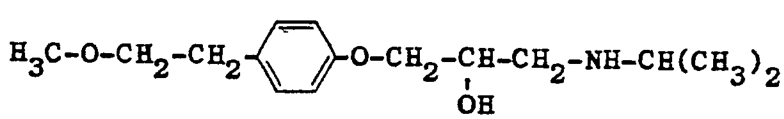

1. Wirkung auf den Kreislauf des narkotisierten Hundes

1.1. β-Receptoren-Blockade

Am pentobarbital-narkotisierten Hund hemmt die erfindungsgemäße Verbindung den Isoprenalinbedingten Kontraktilitäts- und Herzfrequenzanstieg mit einer $ED_{50}$ von 0,003 mg/kg i. v., während der entsprechende Wert für Metoprolol 0,14 mg/kg beträgt. für Metoprolol 0,14 mg/kg beträgt. Die erfindungsgemäße Verbindung ist demnach am Herzen ca. 40 mal wirksamer als Metoprolol (und 11 mal stärker als Propranolol).

Der Vergleich der Hemmung der kardialen β-Receptoren mit der der Blutgefäße $ED_{50}$ (Herz) : $ED_{50}$ (Gefäß), welcher als Maß für die Kardioselektivität anzusehen ist, ergab für die erfindungsgemäße Verbindung ein Verhältnis von 1 : 76 und für Metoprolol von 1 : 18. Die erfindungsgemäße Verbindung wirkt demnach deutlich kardioselektiver.

1.2. Hämodynamik

Das hämodynamische Wirkprofil der erfindungsgemäßen Verbindung am pentobarbital-narkotisierten Hund ist charakterisiert durch einen deutlichen Abfall des Blutdruckes (− 35 mm Hg bei 0,05 mg/kg i. v., Metoprolol − 5 mm Hg), des linksventrikulären enddiastolischen Druckes (LVEDP − 2 mm Hg, Metoprolol + 2 mm Hg) und des totalen peripheren Widerstandes (TPR − 1 191 dyn. sec. $cm^{-5}$, Metoprolol − 135 dyn. sec. $cm^{-5}$) ohne von einer Verminderung der Kontraktilität des Herzens begleitet zu sein, welche bei Metoprolol besonders in höheren Dosen ausgeprägt vorhanden ist.

Ein Absinken der Kontraktilität und der Herzleistung wird bei der erfindungsgemäßen Verbindung durch eine mäßige intrinsische sympathomimetische Aktivität (ISA), welche etwa 1/3 der des Pindolols beträgt, verhindert.

Die erfindungsgemäße Verbindung hat somit ein günstigeres hämodynamisches Wirkprofil als Metoprolol, da es einerseits über eine Senkung des TPR die Nachlast und andererseits auch die Vorlast des Herzens vermindert, ohne negativ inotrop zu wirken.

2. Wirkung am wachen renal-hypertonen Hund

Metoprolol besitzt in der Dosis 3,0 mg/kg p. o. praktisch keine akute blutdrucksenkende Wirkung am wachen renalhypertonen Hund ($\Delta BD_s$ − 6 mm Hg, $\Delta BD_d$ − 3 mm Hg), während die erfindungsgemäße Verbindung bereits in der Dosis 0,2 mg/kg p. o. den systolischen Blutdruck um − 28 mm Hg und den diastolischen um − 15 mm Hg vermindert.

Durch Prüfung in analoger Weise ergeben sich für weitere erfindungsgemäße Verbindungen und für eine aus der DE-A-2 819 629 bekannte, strukturell vergleichbare Verbindung der Formel

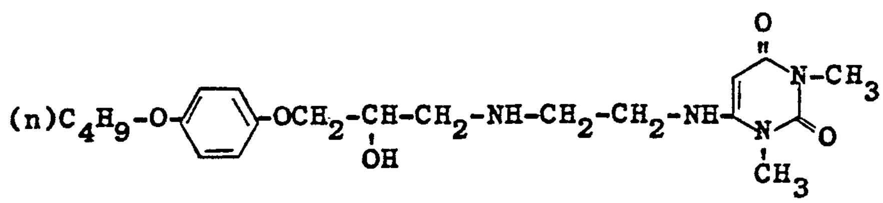

Die aus folgender Tabelle ersichtlichen pharmakologischen Wirkungsdaten :

(Siehe Tabelle, Seite 17 ff)

| $R^1$ | $R^2$ | $R^3$ | $R^4$ | X | $\beta_1$-Blockade $ED_{50}$ mg/kg | Kardioselectivität $\frac{ED_{50}\ Herz}{ED_{50}\ Gefäß}$ | LVEDP Dosis mg/kp | $\Delta$ p [mbar] | TPR Dosis | / $\left[\frac{dyn\ cm}{sec^5}\right]$ |
|---|---|---|---|---|---|---|---|---|---|---|
| 2-Cl | H | H | H | Cl | 0,05 i.d.<br>0,003 i.d. | $\frac{1}{76}$ | 0,05 i.v. | -2,7 | 0,05 | -1191 |
| 2-$OC_4H_9$ | H | H | H | Cl | 0,008 i.v. | $\frac{1}{30}$ | 0,05 i.v. | -1,3 | 0,05 | - 530 |
| 2-$OC_4H_9$ | H | H | -$CH_3$ | Cl | 0,007 i.v. | $\frac{1}{30}$ | 0,05 i.v. | - 2 | 0,05 | - 620 |
| 2-Cl | H | H | H | Br | 0,0008 i.v. | $\frac{1}{81}$ | | | | |
| | | | | | 0,0023 i.v. | $\frac{1}{127}$ | | | | |
| 2-$OC_2H_5$ | H | H | H | Br | 0,0049 i.v. | $\frac{1}{20}$ | | | | |
| H | H | H | H | Br | 0,0009 i.v. | $\frac{1}{743}$ | 0,05 | -5,33 | | |
| H | H | H | H | H | 0,009 i.v. | $\frac{1}{4}$ | | | | |
| Metoprolol | | | | | 0,3 i.d.<br>0,14 i.v. | $\frac{1}{18}$ | | +2,7 | 0,05 | - 135 |
| Verbindung bek. aus DE-A-28 19 629 | | | | | 0,0096 i.v. | | 4,0 i.v. | 0 | 0,05 | 0 |

| $R^1$ | $R^2$ | $R^3$ | $R^4$ | X | Dosis mg/kg | | Blutdruck $/\Delta P_s/\Delta P_d$ [mbar] | | ISA | k-Strophanthin Arrhythmien-Hemmung |
|---|---|---|---|---|---|---|---|---|---|---|
| 2-Cl | H | H | H | Cl | 0,2 | p.o. | -37 | -20 | $\frac{1}{3}$ | + |
| 2-$OC_4H_9$ | H | H | H | Cl | 1 | p.o. | -53 | -40 | | + |
| 2-$OC_4H_9$ | H | H | -$CH_3$ | Cl | 0,1 | p.o. | -33,3 | -26,7 | | + |
| 2-Cl | H | H | H | Br | 0,01 | i.v. | -20 | -20 | | |
| 2-$OC_2H_5$ | H | H | H | Br | 0,001 | i.v. | -40 | -6,7 | | |
| H | H | H | H | Br | 0,5 | i.v. | -100 | -66,7 | | |
| | | | | | 0,025 | i.v. | -40 | -33,3 | | |
| | | | | | 0,064 | i.v. | -53,3 | -40 | | |
| H | H | H | H | H | 0,04 | i.v. | -33,3 | -20 | $\frac{1}{3}$ | |
| | | | | | 0,01 | i.v. | -26,7 | -20 | $\frac{1}{3}$ | |
| | | | | | 0,004 | i.v. | -33,3 | -26,7 | - | |
| Metoprolol | | | | | 3,0 | | 0 | 0 | 0 | 0 |
| Verbindung bek. aus DE-A-28 19 629 | | | | | 4,0 | i.v. | 0 | 0 | 0 | 0 |

Erläuterungen : i. v. = intravenös i. d. = intraduodenal
p. o. = per os, oral ED = Effektivdosis

Die in obiger Tabelle angegebenen Werte der intrinsischen sympathomimetischen Aktivität (ISA) sind relativ zu dem als Standard geltenden Pindolol angegeben.

Die pharmakologischen Werte zeigen, daß die erfingungsgemäßen Verbindungen sich gegenüber den nächstvergleichbaren bekannten Verbindungen durch ein besonders ausgewogenes Wirkungsspektrum auszeichnen. Neben der gegenüber den aus der DE-A-2 819 629 bekannten Verbindungen um mindestens 100 % verbesserten Wirksamkeit bezüglich $\beta_1$-Blockade sind es die günstige, akute blutdrucksenkende Wirkung und insbesondere die sehr ausgewogene intrinsische sympathomimethische Aktivität (ISA), die einerseits ein Absinken der Kontraktilität verhindert, andererseits aber noch nicht zu einem unerwünschten Anstieg der Herzfrequenz führt, die den Einsatz der erfindungsgemäßen Verbindungen besonders vorteilhaft erscheinen lassen.

Ein weiterer Vorteil der erfindungsgemäßen Verbindungen, insbesondere gegenüber den aus der DE-A-2 819 629 bekannten, ist ihre bessere Gewebeverträglichkeit. Dadurch werden beispielsweise bei i. v.-Applikation lokale Reizerscheinungen weitgehend vermieden.

Aufgrund der genannten vorteilhaften Wirkungskombination sind die erfindungsgemäßen Verbindungen besonders geeignet zur Therapie und Prophylaxe des Myocardinfarkts.

Die Tagesdosis für Erwachsene liegt hierbei im Bereich von 5-30 ml als Einzeldosis oder auch verteilt auf 2-3 Einzeldosen.

Die Applikation erfolgt vorzugsweise peroral oder intravenös.

## Ansprüche

1. Basisch substituierte Pyridazine der allgemeinen Formel I

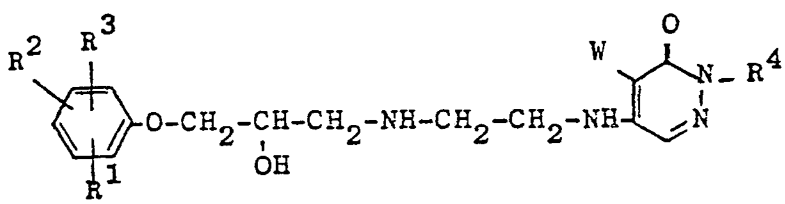

worin $R^1$, $R^2$ und $R^3$ unabhängig voneinander Wasserstoff, Fluor, Chlor, Brom, Hydroxy, Nitro, Trifluormethyl, Alkyl mit 1 bis 8 C-Atomen, Alkoxyalkyl mit 2 bis 6 C-Atomen, Alkenyl mit bis zu 6 C-Atomen, Alkinyl mit bis zu 6 C-Atomen, Cycloalkyl mit einer Ringgröße von 5 bis 8 C-Atomen, Cycloalkenyl mit einer Ringgröße von 5 bis 8 C-Atomen, Phenyl, Alkoxy mit 1 bis 8 C-Atomen, Hydroxyalkoxy mit 2 bis 6 C-Atomen, Alkoxy-alkoxy mit insgesamt bis zu 8 C-Atomen, Alkenyloxy mit bis zu 6 C-Atomen, Alkinyloxy mit bis zu 6 C-Atomen, Cycloalkoxy mit einer Ringgröße von 5 bis 8 C-Atomen, Benzyloxy, Phenethyloxy, Alkanoyl mit 1 bis 6 C-Atomen, Acylamino mit bis zu 11 C-Atomen im Acylrest, einen —NH—CO—$R^9$-Rest, wobei $R^9$ für Morpholino, Piperidino oder 1-Pyrrolidinyl steht, Ureido, Ureido in 3-Stellung monosubstituiert durch Cycloalkyl mit 5 oder 6 C-Atomen, Ureido in 3-Stellung mono- oder disubstituiert durch Alkyl mit 1 bis 6 C-Atomen und/oder Alkenyl mit 3 bis 6 C-Atomen bedeuten, und $R^4$ Wasserstoff oder Alkyl mit 1 bis 4 C-Atomen und W Wasserstoff, Chlor oder Brom bedeuten, sowie deren Saüreadditionssalze.

2. Verbindungen nach Anspruch 1, dadurch gekennzeichnet, daß nur einer der Substituenten $R^1$, $R^2$ oder $R^3$ ungleich Wasserstoff ist.

3. Verbindungen nach den Ansprüchen 1 bis 2, dadurch gekennzeichnet, daß $R^4$ Wasserstoff ist.

4. Verbindungen nach den Ansprüchen 1 bis 3, dadurch gekennzeichnet, daß einer der Substituenten $R^1$, $R^2$ oder $R^3$ Halogen und die beiden anderen Wasserstoff bedeuten.

5. Die Verbindung der Formel

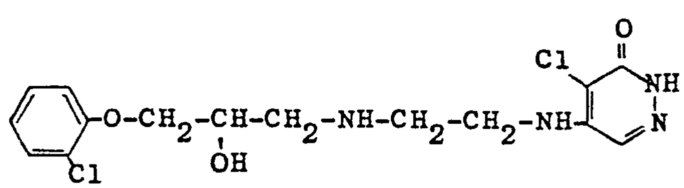

und ihre pharmazeutisch annehmbaren Säureadditionssalze.

6. Verfahren zur Herstellung basisch substituierter Pyridazine der allgemeinen Formel I gemäß Anspruch 1, dadurch gekennzeichnet, daß

a) eine Verbindung der allgemeinen Formel V

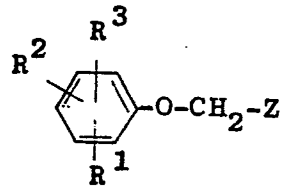

(V)

worin Z für

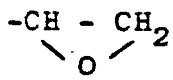 oder 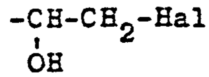

steht und Hal Halogen bedeutet, mit einer Verbindung der allgemeinen Formel IV

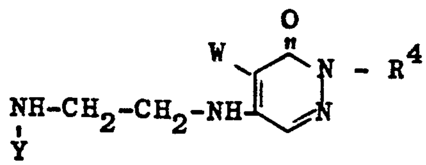

(IV)

wobei $R^1$, $R^2$, $R^3$, $R^4$ und W die obengenannten Bedeutungen besitzen und wobei Y für Wasserstoff oder einen hydrogenolytisch abspaltbaren Rest steht, umgesetzt und aus der entstandenen Verbindung ein hydrogenolytisch abspaltbarer Rest Y durch Hydrogenolyse abgespalten und die erhaltene Verbindung gegebenfalls mit einer Säure zu einem Säureadditionssalz umgesetzt wird oder

b) eine Verbindung der allgemeinen Formel VI

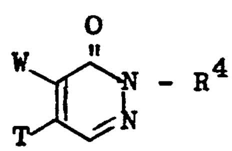

(VI)

worin $R^4$ und W die obengenannten Bedeutungen haben und T für Chlor oder Brom steht mit einer Verbindung der allgemeinen Formel IX

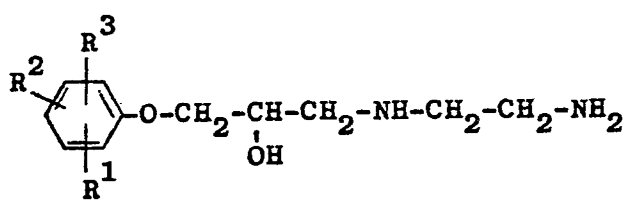

(IX)

worin $R^1$, $R^2$, $R^3$ die obengenannten Bedeutungen besitzen, um gesetzt wird und die erhaltene Verbindung gegebenenfalls mit einer Säure zu einem Säureadditionssalz umgesetzt wird.

7. Verfahren gemäß Anspruch 6, dadurch gekennzeichnet, daß W Chlor oder Brom ist.

8. Verfahren zur Herstellung basisch substituierter Pyridazine der allgemeinen Formel I, in denen W Wasserstoff ist und $R^1$, $R^2$, $R^3$ und $R^4$ die in Anspruch 1 genannten Bedeutungen haben, dadurch gekennzeichnet, daß man ein basisch substituiertes Pyridazin der allgemeinen Formel Ia

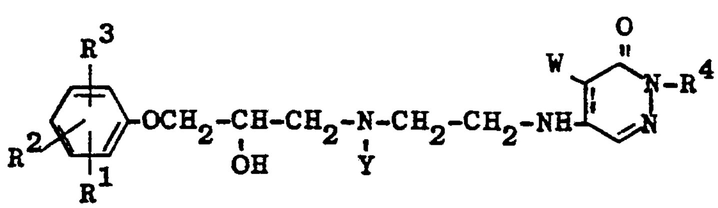

(Ia)

worin $R^1$, $R^2$, $R^3$ und $R^4$ die in Anspruch 1 angegebenen Bedeutungen haben und W Chlor oder Brom und Y Wasserstoff oder ein hydrogenolytisch abspaltbarer Rest ist, in an sich bekannter Weise in einem Lösungs- oder Dispergiermittel in Gegenwart eines Hydrierkatalysators mit Wasserstoff hydriert.

9. Verfahren gemäß Anspruch 8, dadurch gekennzeichnet, daß eine Verbindung der Formel Ia eingesetzt wird, in der Y Wasserstoff ist.

10. Pharmazeutische Zubereitung, enthaltend eine wirksame Dosis eines basisch substituierten Pyridazins der allgemeinen Formel I oder ein Säureadditionssalz davon, hergestellt nach einem der Ansprüche 3 bis 9, neben pharmazeutisch zulässigen Träger- und Zusatzstoffen.

11. Pharmazeutische Zubereitung nach Anspruch 10, enthaltend zusätzlich noch eine oder mehrere andere pharmazeutisch wirksame Substanzen.

**Claims**

1. Basically substituted pyridazines of the general formula I

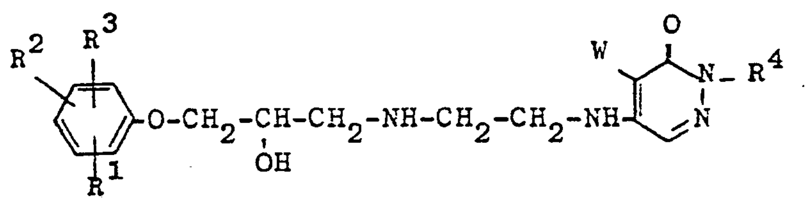

(I)

wherein $R^1$, $R^2$ and $R^3$ independently from one another denote hydrogen ; fluorine ; chlorine ; bromine ; hydroxyl ; nitro ; trifluoromethyl ; alkyl having 1 to 8 C atoms ; alkoxyalkyl having 2 to 6 C atoms ; alkenyl having up to 6 C atoms ; alkynyl having up to 6 C atoms ; cycloalkyl having a ring size of from 5 to 8 C atoms ; cycloalkenyl having a ring size of from 5 to 8 C atoms, phenyl ; alkoxy having 1 to 8 C atoms ; hydroxyalkoxy having 2 to 6 C atoms ; alkoxyalkoxy having a total of up to 8 C atoms ; alkenyloxy having up to 6 C atoms ; alkynyloxy having up to 6 C atoms ; cycloalkoxy having a ring size of from 5 to 8 C atoms ; benzyloxy ; phenethoxy ; alkanoyl having 1 to 6 C atoms ; acylamino having up to 11 C atoms in the acyl radical ; an —NH—CO—$R^9$ radical, $R^9$ denoting morpholino, piperidino or 1-pyrrolidinyl ; ureido ; ureido which is monosubstituted in the 3-position by cycloalkyl having 5 or 6 C atoms ; ureido which is monosubstituted or disubstituted in the 3-position by alkyl having 1 to 6 C atoms and/or alkenyl having 3 to 6 C atoms ; $R^4$ denotes hydrogen or alkyl, having 1 to 4 C atoms and W denotes hydrogen, chlorine or bromine and acid-addition salts thereof.

2. Compounds of Claim 1, characterised in that only one of the substituents $R^1$, $R^2$ and $R^3$ is not hydrogen.

3. Compounds of Claims 1 to 2, characterised in that $R^4$ is hydrogen.

4. Compounds of Claims 1 to 3, characterised in that one of the substituents $R^1$, $R^2$ and $R^3$ is halogen and the other two are hydrogen.

5. The compound of the formula

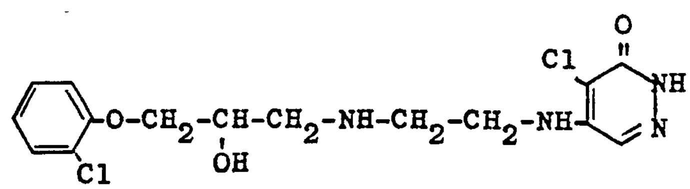

and pharmaceutically acceptable acid addition salts thereof.

6. Process for the preparation of basically substituted pyridazines of the general formula I of Claim 1, characterised in that

a) a compound of the general formula V

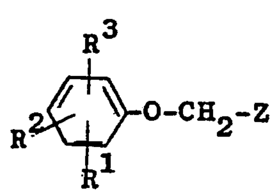

(V)

wherein Z denotes

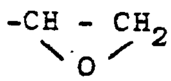 or 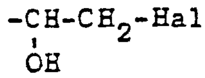

and Hal denotes halogen is reacted with a compound of the general formula IV

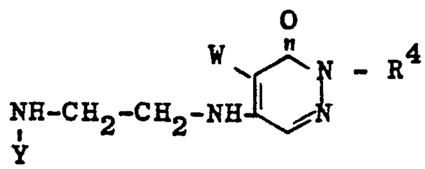

(IV)

wherein $R^1$, $R^2$, $R^3$, $R^4$ and W have the meanings indicated above ans Y is hydrogen or a radical capable of being hydrogenolytically split off and a radical Y which can be hydrogenolytically split off is split off by hydrogenolysis from the compound obtained, and the compound obtained is, if appropriate, reacted with an acid to give an acid addition salt, or

b) a compound of the general formula VI

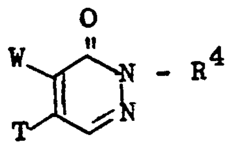

(VI)

wherein $R^4$ and W have the above meanings and T is chlorine or bromine is reacted with a compound of the general formula IX

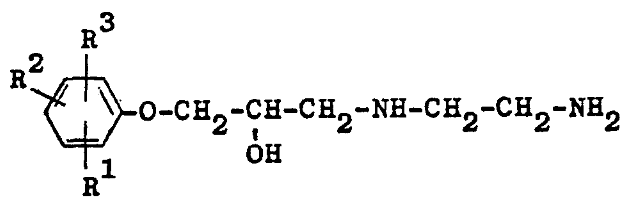

(IX)

wherein $R^1$, $R^2$ and $R^3$ have the above meanings and the compound obtained is, if appropriate, reacted with an acid to give an acid addition salt.

7. The process of Claim 6, characterised in that W is chlorine or bromine.

8. Process for the preparation of basically substituted pyridazines of the general formula I wherein W is hydrogen and $R^1$, $R^2$, $R^3$ and $R^4$ have the meanings indicated in Claim 1, characterised in that a basically substituted pyridazine of the general formula Ia

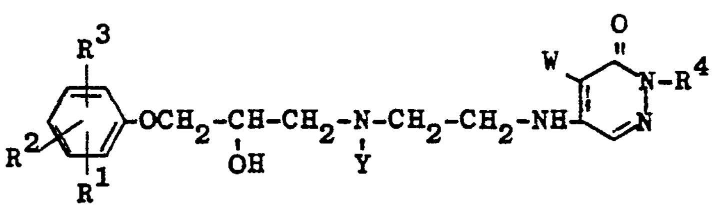

(Ia)

wherein $R^1$, $R^2$, $R^3$ and $R^4$ have the meanings indicated in Claim 1 and W is chlorine or bromine and Y is hydrogen or a radical which can be split off, is hydrogenated with hydrogen in a manner known per se in a solvent or dispersing agent and in the presence of a hydrogenation catalyst.

9. The process of Claim 8, characterised in that a compound of formula Ia in which Y is hydrogen is employed.

10. Pharmaceutical preparation containing an effective dose of a basically substituted pyridazine of the general formula I or an acid addition salt thereof, prepared according to one of Claims 6 to 9, in addition to pharmaceutically permissible excipients and additives.

11. The pharmaceutical preparation of Claim 10, additionally containing one or several other pharmaceutically effective substances.

**Revendications**

1. Pyridazines à substitution basique, de formule générale I

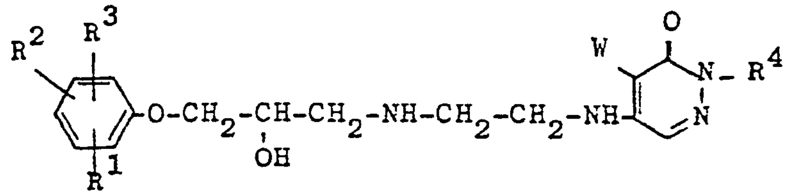

dans laquelle $R^1$, $R^2$ et $R^3$ représentent chacun, indépendamment l'un de l'autre, un atome d'hydrogène, de fluor, de chlore ou de brome ou un groupe hydroxyle, nitro, trifluorométhyle, alkyle ayant 1 à 8 atomes de carbone, alcoxyalkyle ayant 2 à 6 atomes de carbone, alcényle ayant jusqu'à 6 atomes de carbone, alcynyle ayant jusqu'à 6 atomes de carbone, cycloalkyle dont le cycle comporte 5 à 8 atomes de carbone, cycloalcényle dont le cycle comporte 5 à 8 atomes de carbone, phényle, alcoxy ayant 1 à 8 atomes de carbone, hydroxyalcoxy ayant 2 à 6 atomes de carbone, alcoxyalcoxy ayant au total jusqu'à 8 atomes de carbone, alcynyloxy ayant jusqu'à 6 atomes de carbone, alcényloxy ayant jusqu'à 6 atomes de carbone, cycloalcoxy dont le cycle comporte 5 à 8 atomes de carbone, benzyloxy, phénétyloxy, alcanoyle ayant 1 à 6 atomes de carbone, acylamino ayant jusqu'à 11 atomes de carbone dans le radical acyle, un radical —NH—CO—$R^9$, dans lequel $R^9$ représente un cycle morpholino, pipéridino ou pyrrolidinyle-1, un radical uréido, uréido monosubstitué en position 3 par un groupe cycloalkyle ayant 5 ou 6 atomes de carbone, uréido mono- ou disubstitué en position 3 par un groupe alkyle ayant 1 à 6 atomes de carbone et/ou alcényle ayant 3 à 6 atomes de carbone, et $R^4$ représente de l'hydrogène ou un groupe alkyle ayant 1 à 4 atomes de carbone et W de l'hydrogène, du chlore ou du brome, ainsi que leurs sels d'addition d'acides.

2. Composés selon la revendication 1, caractérisés en ce qu'un seul substituant $R^1$, $R^2$ et $R^3$ n'est pas de l'hydrogène.

3. Composés selon les revendications 1 et 2, caractérisés en ce que $R^4$ est de l'hydrogène.

4. Composés selon les revendications 1 à 3, caractérisés en ce que l'un des substituants $R^1$, $R^2$ et $R^3$ représente un halogène et les deux autres de l'hydrogène.

5. Composé de formule

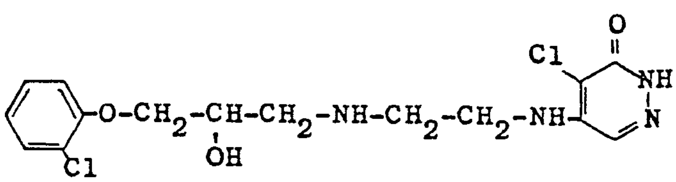

et ses sels d'addition d'acides acceptables du point de vue pharmaceutique.

6. Procédé de préparation de pyridazines à substitution basique, de formule générale I selon la revendication 1, procédé caractérisé en ce que

a) on fait réagir un composé de formule générale V

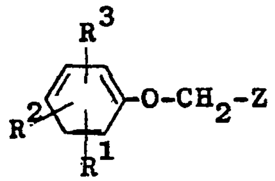

(V)

dans laquelle Z représente

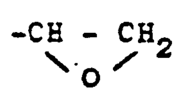

ou

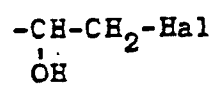

et Hal représente un halogène avec un composé de formule générale IV

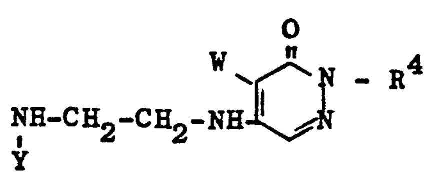

(IV)

dans laquelle $R^1$, $R^2$, $R^3$, $R^4$ et W ont les sens indiqués ci-dessus, et Y représente un atome d'hydrogène ou un radical pouvant être coupé ou séparé par hydrogénolyse et à partir du composé résultant, on coupe par hydrogénolyse un radical Y pouvant être enlevé par hydrogénolyse, et l'on fait éventuellement réagir le composé obtenu avec un acide pour obtenir un sel d'addition d'acide, ou

b) on fait réagir un composé de formule générale VI

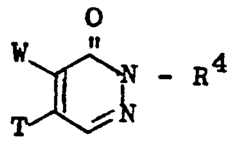

(VI)

dans laquelle $R^4$ et W ont les sens indiqués ci-dessus, et T représente du chlore ou du brome avec un composé de formule générale IX

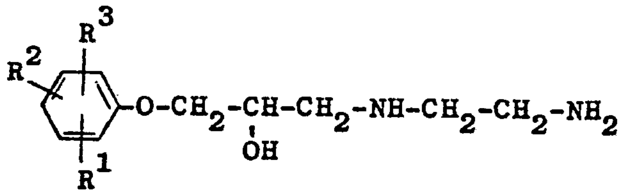

(IX)

dans laquelle $R^1$, $R^2$, $R^3$ ont les sens précipités et l'on fait éventuellement réagir le composé ainsi obtenu avec un acide pour obtenir un sel d'addition d'acide.

7. Procédé selon la revendication 6, caractérisé en ce que W représente du chlore ou du brome.

8. Procédé de préparation de pyridazines à substitution basique de formule générale I (dans laquelle W représente de l'hydrogène et $R^1$, $R^2$, $R^3$ et $R^4$ ont les sens indiqués à la revendication 1), caractérisé en ce qu'on hydrogène avec de l'hydrogène, en présence d'un catalyseur d'hydrogénation, une pyridazine à substitution basique de formule générale Ia

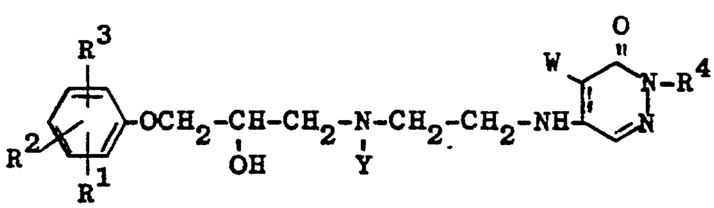

dans laquelle $R^1$, $R^2$, $R^3$ et $R^4$ ont les sens indiqués à la revendication 1, et W représente le chlore ou le brome et Y représente l'hydrogène ou un radical pouvant être séparé ou coupé par hydrogénolyse en opérant de façon connue en soi dans un solvant ou milieu de dispersion.

9. Procédé selon la revendication 8, caractérisé en ce qu'on utilise un composé de formule Ia, dans laquelle Y est de l'hydrogène.

10. Préparation pharmaceutique, contenant une dose efficace d'une pyridazine à substitution basique de formule générale I ou d'un sel d'addition d'acide de ce composé, préparée selon l'une des revendications 6 à 9, en plus de supports, véhicules et additifs pharmaceutiquement acceptables.

11. Préparation pharmaceutique selon la revendication 10, contenant en outre encore une ou plusieurs autres substances à activité pharmaceutique.